(19) Europäisches Patentamt
European Patent Office
Office européen des brevets

(11) Publication number: 0 280 239 B1

# (12) EUROPEAN PATENT SPECIFICATION

(45) Date of publication of the patent specification: 04.07.90

(51) Int. Cl.[5]: C07D 211/90, C07D 401/12, A61K 31/44

(21) Application number: 88102577.9

(22) Date of filing: 22.02.88

(54) 1,4-Dihydropyridine derivatives, production and use thereof.

(30) Priority: 24.02.87 JP 39047/87

(43) Date of publication of application: 31.08.88 Bulletin 88/35

(45) Publication of the grant of the patent: 04.07.90 Bulletin 90/27

(84) Designated Contracting States: CH DE FR GB IT LI

(56) References cited:
EP-A- 0 174 131
EP-A- 0 220 917

(73) Proprietor: SANWA KAGAKU KENKYUSHO CO., LTD., No. 35, Higashi-sotobori-cho, Higashi-ku Nagoya-shi Aichi-ken(JP)

(72) Inventor: Kurono, Masayasu, 3-6-7, Sasao-nishi Toin-cho, Inabe-gun Mie-ken(JP)
Inventor: Suzuki, Tsunemasa, 666 Minami-toyozaki Matsubase-machi, Shimomashiki-gun Kumamoto-ken(JP)
Inventor: Kondo, Yasuaki, 5-56, Shinogi-cho, Kasugai-shi Aichi-ken(JP)
Inventor: Suzuki, Tomoo, 7-401, 6-2-3, Iwanari-dai, Kasugai-shi Aichi-ken(JP)
Inventor: Sawai, Kiichi, 1-36-14, Ninomiya, Funabashi-shi Chiba-ken(JP)

(74) Representative: Wächtershäuser, Günter, Dr., Tal 29, D-8000 München 2(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The present invention relates to novel 1,4-dihydropyridine dearivatives, salts thereof, a process for the preparation of same, and a pharmaceutical composition comprising the derivative or salt.

It has been well known that certain kind 1,4-dihydropyridine derivatives show a powerful calcium (Ca) antagonistic effect and thus can be employed as an effective ingredient for a pharmaceutical composition, namely the medicine for curing blood circulatory diseases.

As one of exemplar known, 1,4-dihydropyridine derivatives, dimethyl ester of 1,4-dihydro-2,6-dimethyl-4-(2-nitrophenyl)pyridine-3,5-dicarboxylic acid (known as --Nifedipine--) may be listed, which shows a colonary vasodilating action, blood pressure depressing action and the like [W. Vater et al. "Arzneim. - Forsch" (Drug Res.), Vol. 22, page 1, (1972)]. As another exemplar compound, there is 1,4-dihydro-2,6-dimethyl-4-(3-nitrophenyl)pyridine-3,5-dicarboxylic acid 3-2-(N-benzyl-N-methylamino)ethyl 5-methyl ester (known as --Nicardipine--), which shows a cerebral blood flow increasing action.

Among the both of such known compounds, the Nifedipine has no action for increasing cerebral blood flow and shows a powerful blood flow depressing action, which causes a disadvantage of a relative decrease on the cerebral blood flow. On the other hand, the Nicardipine having a higher cerebral bood increasing action in comparison with the Nifedipine, has been developed and actually employed as one of cerebral circulation improving agents, but it can not be said that it selectively acts to the cerebral blood path to increase only the blood flow therein.

Therefore, a principal object of the invention is to provide novel 1,4-dihydropyridine derivatives and salts thereof, which has an excellent Ca antagonistic action and acts selectively to cerebral blood path to show a powerful cerebral blood increasing action.

Additional objects of the invention are to provide a process for the manufacture of such derivates and salts as well as a pharmaceutical composition containing at least one of such derivates and salts, as an effective ingredient therefor.

According to the invention, the principal object is attained by a 1,4-dihydropyridine derivative of the formula

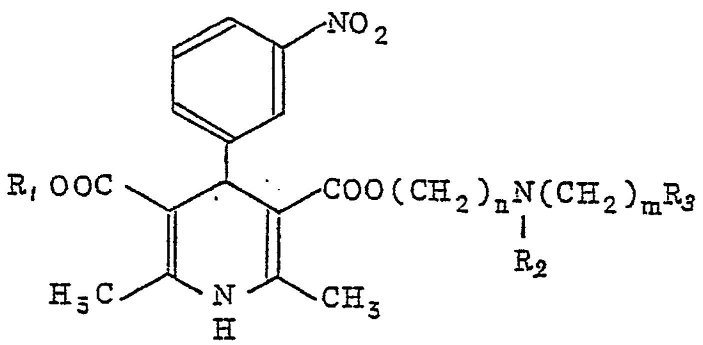

(I)

wherein

$R_1$ is cyclopropylmethyl, $R_2$ is methyl, $R_3$ is 2,4-dichlorophenyl, n is 2, and m is 1, or

$R_1$ is cyclopropylmethyl, n is 2, m is 1, and $R_2$ and $R_3$ bond together with the neighbouring $-N(CH)_2m$-radical to mean 5-chloroisoindolin-2-yl radical,

or a salt thereof.

According to the process of the invention, the compounds (Formula I) and salts thereof can be prepared by reacting in the presence of ammonia or a salt thereof, a compound of the formula

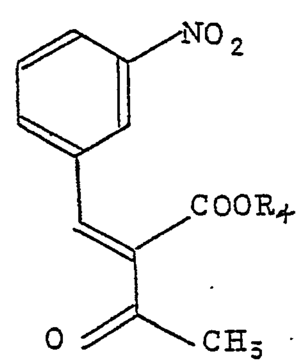

(II)

wherein $R_4$ is cyclopropylmethyl group or a radical of

$$-(CH_2)_n\underset{\displaystyle R_2}{\underset{|}{N}}(CH_2)_mR_3$$

in which $R_2$, $R_3$, n and m have meanings as referred to, with a compound of the formula

$$CH_3\overset{\overset{\displaystyle O}{\|}}{C}CH_2COOR_5 \qquad (III)$$

wherein $R_5$ is cyclopropylmethyl group or the radical of

$$-(CH_2)_n\underset{\displaystyle R_2}{\underset{|}{N}}(CH_2)_mR_3$$

which $R_2$, $R_3$, n and m have meanings as referred to but $R_5$ in this compound (III) and $R_4$ in the compound (II) do not have simultaneously same meaning of the radical of

$$-(CH_2)_n\underset{\displaystyle R_2}{\underset{|}{N}}(CH_2)_mR_3$$

and if necessary, converting resulting reaction product into the salt.

In this case, it is preferable to select a molar ratio of about 1:0.8 to 1:1.5 for the raw materials II and III. The raction can be carried out at a temperature of about 50 to 150°C in the presence or absence of a solvent. As the solvent, methanol, ethanol, isopropanol or the like alcohol; benzene, toluene or the like aromatic hydrocarbon; dimethylformamide or the like aprotic polar solvent; ethyl ether, tetrahydrofuran or the like ether; or water can be employed. A separation or isolation of the desired compound from the reaction mixture is carried out through operations known per se, for instance concentration, extraction, column chromatography, recrystallization and others.

The benzylidene as one of the raw materials and shown by Formula II may be of that obtained from the market or otherwise, can be synthesized by a condensation reaction between an aldehyde of the formula

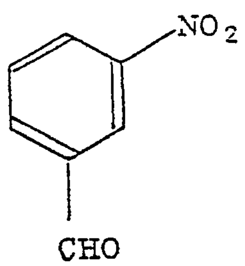

(IV)

and the ester shown by Formula III, in accordance with the method disclosed by G. Jones in "Org. Reactions" Vol. 15, page 204 (1967). On the other hand, the ester as the other raw material and shown by Formula III may also be of that obtained from the market or otherwise, can be synthesized in accordance with the method disclosed by Oren-Olov Lawesson et al. In "Org. Syn." Vol. V, page 155 (1973).

For synthsizing the compound shown by Formula 1, ammonia or salt thereof is employed, and as the salt, ammonium acetate, ammonium carbonate, ammonium hydrogen carbonate or the like can be listed.

The compounds shown by Formula I may be synthesized through various routes other than that as referred to, according to the invention and thus some of which shall be explained below, for mere references.

Route A

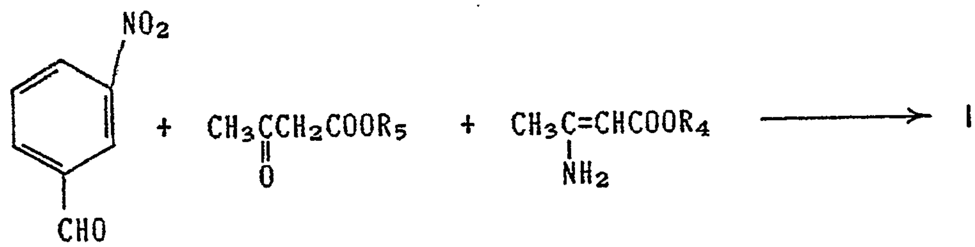

(IV) (III) (V)

wherein $R_4$ and $R_5$ have the meanings as referred to.

The synthetic reaction for this route can be carried out in accordance with the methods disclosed by H. H. Fox et al. in "J. Org. Chem." Vol. 16, page 1259 (1951). It is preferable to select a molar ratio of about 1 : 0.8 : 0.8 to 1 : 1.5 : 1.5 for the raw materials. Reaction conditions, solvent and operations for the reaction are substantially same with those for the process according to the invention.

The aldehyde shown by Formula IV is available in the market, and as to the ester shown by Formula III, please refer to the description given before on the process according to the invention. The enaminoester shown by Formula V can be obtained from the market and otherwise, synthesized in accordance with the method disclosed for instance, by S. A. Glickman and A. C. Cope in "J. Am. Chem. Soc." Vol. 67, page 1017 (1945).

Route B

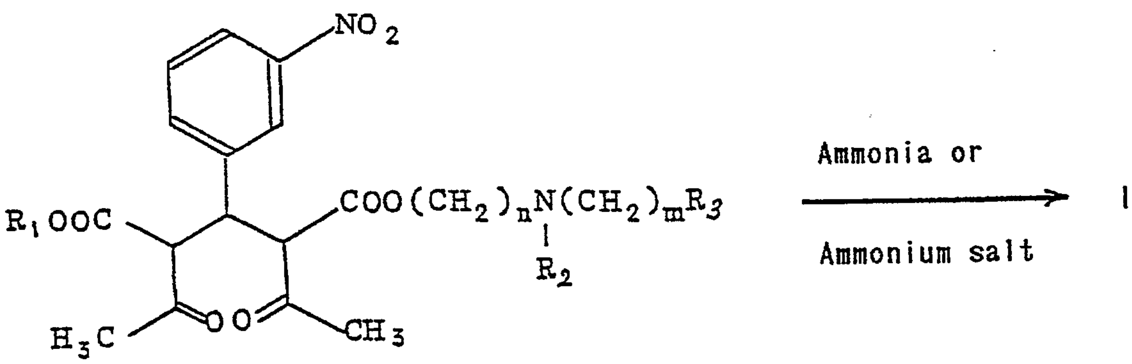


wherein $R_1$, $R_2$, $R_3$, n and m have the meanings as referred to.

This synthetic route is similar to the method disclosed by A. Singer and S. M. McElvain in "Org. Syn." Vol. 11, page 214 (1943), and reaction conditions, solvent and operations are substantially same with those for the process according to the invention.

The diketoester shown by Formula VI may be of that available in the market and otherwise, can be synthesized by treating the compound II as employed for the invention, in the presence of a catalyst of potassium fluoride, cesium fluoride or the like, in accordance with the method disclosed by G. jones in "Org. Reactions" Vol. 15, page 204 (1967).

Route C

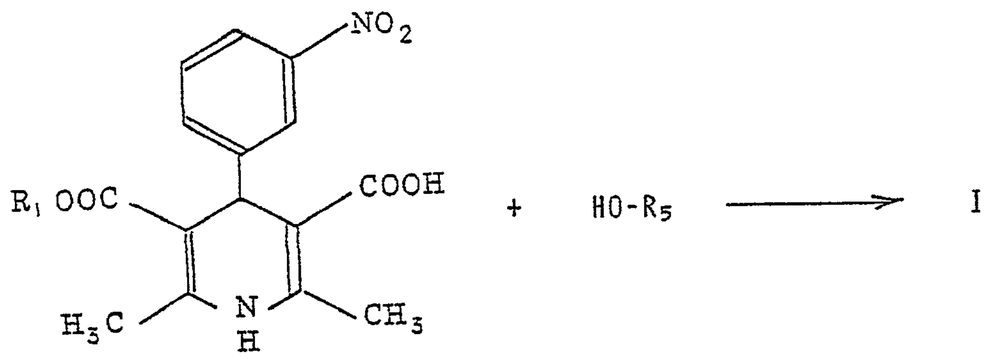

(VII) (VIII)

wherein $R_1$ and $R_5$ have the meanings as referred to.

This synthetic route is similar to that disclosed by T. Shibanuma et al. in "Chem. Pharm. Bull." Vol. 28, page 2809 (1980). The carboxylic acid shown by Formula VII is available in the market or otherwise, can be synthesized in accordance with the method disclosed in the literature.

According to this synthetic route, the raw material shown by Formula VII is reacted firstly with thionyl chloride, phosphorous pentachloride or ethyl chloroformate in equimolar amount, in the presence of triethylamine or pyridine in equimolar amount, in a solvent such as methylene chloride, chloroform or the like halogenated hydrocarbon; benzene, toluene or the like aromatic hydrocarbon; diethyl-ether, tetrahydrofuran, dimethoxyethane, dioxane or the like ether; dimethylformamide or the like aprotic solvent; or any mixture thereof, and then reacted with the alcohol (VIII) in equimolar amount. Otherwise, the compound shown by Formula I can be synthesized by mixing the raw material compounds VII and VIII is equimolar amount, adding to the mixture dicyclohexylcarbodiimide in equimolar amount, and causing a reaction thereof in said solvent and in the presence of N,N-dimethylaminopyridine in a catalytic amount.

Even if any of synthetic methods is selected for this Route C, an isolation of the objective compound from the reaction mixture is carried out in the manner similar to that for the process according to the invention.

Returning now to the invention, each of the derivatives and salts according to the invention shows an excellent Ca antagonistic action and acts selectively to the cerebral blood path to increase a blood flow therein, whereby it is suitable as the ingredient for medicines improving cerebral blood circulation.

While, according to the process of the invention, such useful derivatives and salts can be prepared through easier operations, by starting from the raw materials which are easy available in the market or can easily be synthesized.

The medicine comprising at least one of the derivatives or salts thereof can be prepared in a conventional manner. A form of the medicine may be a tablet, capsule, granule or the like for oral dosage. A dose of the derivative or salt depends on various factors such as kind of the specified compound or salt to be used, selected medicine form, condition of illness and age of a patient and the like, but in case of an adult, 1 to 100mg/day is generally preferable.

The invention will now be further explained with reference to Examples for preparing compounds, Pharmacological Test Examples and Examples for preparing medicines.

Example 1

3-Cyclopropylmethyl 5-[2-[N-(2,4-dichlorobenzyl)-N-methylamino]-ethyl]1,4-dihydro-2,6-dimethyl-4-(3-nitrophenyl)-pyridine-3,5-dicarboxylate

A mixture of 15.1g (47.5mmol) of 2-[2,4-dichlorobenzyl)-N-methylamino]ethyl acetoacetate, 13.7g (47.4mmol) of cyclopropylmethyl 2-(3-nitrobenzylidene)acetoacetate, 3.4ml of 28% aqueous ammonia and 70ml of ethanol was stirred at its reflux temperature for 7 hours, and then the solvent was removed in vacuo.

The resulting residue was chromatographed on silica gel column to afford 17.4g (62.4%) of the desired compound as yellow oil. Treatment of this oil with hydrochloric acid gave corresponding salt.

| | |
|---|---|
| Melting point: 95–101°C | |
| Elementary analysis ($C_{29}H_{31}Cl_2N_3O_6$ HCl · $H_2O$): | |
| Cal.; H 5.33, C 54.17, N 6.54 | |
| Found; H 5.08, C 53.93, N 6.53 | |
| Mass spectrum (EI/DI) m/z: | |
| 587 ($M^+$), 180 (base peak) | |
| $^1$H-NMR spectrum (DMSO-$d_6$) δppm: | |
| 0.00–1.40 | (5H, m) |
| 2.33 | (3H, s) |
| 2,41 | (3H, s) |
| 2.69 | (3H, s) |
| 3.35–3.70 | (2H, m) |
| 3.87 | (2H, d) |
| 4.27–4.67 | (2H, m) |
| 5.10 | (1H, s) |
| 7.47–8.23 | (2H, m) |
| 9.48 | (1H, brs) |

Example 2

3-[2-(5-Chloroisoindolin-2-yl)ethyl] 5-cyclopropymethyl 1.4-dihydro-2,6-dimethyl-4-(3-nitrophenyl)-pyridine-3,5-dicarboxylate

A mixture of 9.95g (35.4mmol) of 2-(5-chloroisoindolin-2-yl)ethyl acetoacetate, 10.2g (35.3mmol) of cyclopropylmethyl 2-(3-nitro benzylidene)acetoacetate, 2.6ml of 28% aqueous ammonia and 60 ml of ethanol was stirred at its reflux temperature for 7 hours, and then the solvent was removed in vacuo.

The resulting residue was chromatographed on silica gel column and crystalized from methanol to afford 9.9g (50.8%) of the desired compound as yellow crystals.

| | |
|---|---|
| Melting point: 145–148°C | |
| Elementary analysis ($C_{29}H_{30}ClN_3O_6$): | |
| Cal.; H 5.48, C 63.10, N 7.61 | |
| Fount; H 5.49, C 62.81, N 7.54 | |
| Mass spectrum (EI/DI) m/z: | |
| 551 ($M^+$), 189 (base peak) | |
| $^1$H-NMR spectrum ($CDCl_3$) δppm: | |
| 0.00–1.37 | (5H, m) |
| 2.38 | (6H, s) |
| 2.96 | (2H, t) |
| 3.88 | (2H, d) |
| 3.92 | (4H, s) |
| 4.25 | (2H, t) |
| 5.20 | (1H, s) |
| 6.03 | (1H, brs) |
| 7.13–8.23 | (7H, m) |

Pharmacological Test Example 1 (Blood flow increasing action)

An experiment was carried out by anesthetizing adult made and female mongrel dogs (weight ; 10 to 20kg) with Nembutal (30mg/kg, i.p.) and artificially respiring (20ml/kg/stroke, 20 strokes/min.) the animals.

Right vertebral and femoral arteries were exposed and each probe of electro-magnetic flow meter was fitted therein to measure vertebral and femoral arterial blood flows. A cannula was inserted into descending aorta from left femoral artery, in order to measure blood pressure by a pressure transducer connected to an amplifier. Each of test compounds dissolved in 50% ethanol solution (1μg/kg, i.v.) was administered from the cannula into cephlic vein.

Results of change in blood flow and blood pressure are given in following Table 1.

Table 1

| Compound | Number of head | Change (%) VABF | FABF | DBP |
|---|---|---|---|---|
| Test | | | | |
| A | 5 | 90 ± 17 | 23 ± 7 | −7 ± 1 |
| B | 5 | 40 ± 6 | 23 ± 8 | −7 ± 2 |
| Control | | | | |
| Nicardipine | 5 | 32 ± 7 | 14 ± 5 | −10 ± 1 |

In the Table
A: Compound of Example 1 (hydrochloric acid salt).
B: Compound of Example 2
VABF: Vertebral arterial blood flow,
FABF: Femoral arteial blood flow, and
DBP: Diastolic blood pressure.

Further, an amount of the compound required to increase the vertebral arterial blood flow by 50% was checked to obtain results shown in following Table 2, wherein the amount is given as $ED_{50}$ (μg/kg).

Table 2

| Compound | $ED_{50}$, i.v. |
|---|---|
| Example | |
| 1 | 1.1 |
| 2 | 1.6 |
| Nicardipine | 4.2 |

Pharmacological Test Example 2 (Blood pressure depressing action)

To male spontaneously hypertensive rats (15 or more weeks old), each of test compounds was orally administered and then measurement of systric pressure of median sacral artery was carried out at the time which lapsed 1, 2, 4, 6, and 24 hours from the administration, to obtain results given in following Table 3.

Table 3

| Compound | Dose (mg/kg) | N | Pre-value (%) | 1 hr (%) | 2 hr (%) | 4 hr (%) | 6 hr (%) | 24 hr (%) |
|---|---|---|---|---|---|---|---|---|
| No compound | – | 6 | 214 ± 2.5 | 0.1 ± 1.25 | 0.7 ± 1.12 | 4.3 ± 1.55 | –1.4 ± 2.94 | –3.3 ± 1.14 |
| Testing | | | | | | | | |
| Example 1 | 3.0 | 6 | 220 ± 2.4 | –33.3 ± 6.57 | –35.2 ± 4.12 | –34.0 ± 2.24 | –32.8 ± 2.72 | –13.0 ± 5.94 |
| Example 2 | 3.0 | 6 | 217 ± 3.1 | –3.0 ± 1.47 | –9.0 ± 2.82 | –13.2 ± 6.15 | –21.4 ± 3.67 | –14.3 ± 4.09 |
| Control | | | | | | | | |
| Nifedipine | 3.0 | 6 | 218 ± 3.0 | –19.3 ± 4.41 | –10.6 ± 3.14 | –4.3 ± 2.70 | –0.1 ± 1.56 | –1.1 ± 1.06 |
| Nicardipine | 3.0 | 6 | 222 ± 1.8 | –10.0 ± 2.28 | –5.7 ± 1.61 | –3.0 ± 2.01 | –1.0 ± 1.50 | –4.2 ± 1.88 |

N: Numer of heads of animals.

Medicine Preparing Example 1 (Tablet)
Following components were mixed to prepare tablets in a conventional manner

| | |
|---|---|
| Compound of Example 1 (hydrochloric acid salt) | 5 (mg) |
| Potato starch | 55 |
| Fine crystalline cellulose | 30 |
| Gelatin | 8 |
| Magnesium stearate | 2 |
| | 100 mg/tablet |

Medicine Preparing Example 2 (Capsule)
Following components were mixed to prepare capsules in a conventional manner

| | |
|---|---|
| Compound of Example 1 (hydrochloric acid salt) | 5 (mg) |
| Corn starch | 106 |
| Lactose | 35 |
| Polyvinylpyrrolidone | 2 |
| Magnesium stearate | 2 |
| | 150 mg/capsule |

## Claims

1. A 1,4-dihydropyridine derivative of the formula

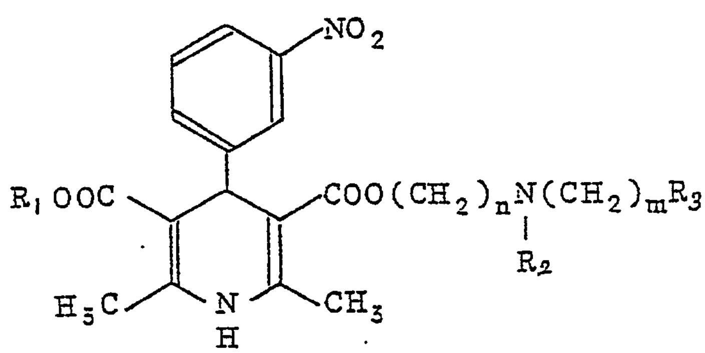

(I)

wherein
$R_1$ is cyclopropylmethyl, $R_2$ is methyl, $R_3$ is 2,4-dichlorophenyl, n is 2, and m is 1, or
$R_1$ is cyclopropylmethyl, n is 2, m is 1, and $R_2$ and $R_3$ bond together with the neighbouring $-N(CH_2)m-$ radical to mean 5-chloroisoindolin-2-yl radical,
or a salt thereof.

2. A process for the preparation of a 1-4-dihydropyridine derivative of the formula

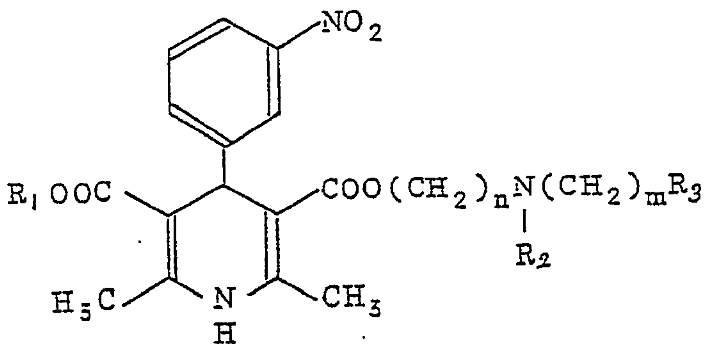

(I)

wherein $R_1$, $R_3$, <u>n</u>, and <u>m</u> are as defined in claim 1, or a salt thereof, which comprises a step of reacting in the presence of ammonia or a salt thereof, a compound of the formula

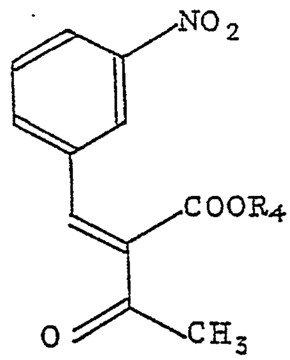

(II)

wherein $R_4$ is a cyclopropylmethyl group or a radical of

$$-(CH_2)n\underset{R_2}{\underset{|}{N}}(CH_2)mR_3$$

in which $R_2$, $R_3$, <u>n</u> and <u>m</u> have meanings as referred to, with a compound of the formula

$$CH_3\overset{O}{\overset{\|}{C}}CH_2COOR_5 \qquad (III)$$

wherein $R_5$ is a cyclopropylmethyl group or the radical of

$$-(CH_2)n\underset{R_2}{\underset{|}{N}}(CH_2)mR_3$$

in which $R_2$, $R_3$, <u>n</u> and <u>m</u> have meanings as referred to, but $R_5$ in this compound (III) and $R_4$ in the compound (II) do not have simultaneously same meaning of the radical of

$$-(CH_2)n\underset{R_2}{\underset{|}{N}}(CH_2)mR_3$$

and if necessary, converting resulting reaction product into the salt.

3. A pharmaceutical composition for improving cerebral blood flow circulation, which comprises an effective amount of 1,4-dihydropyridine derivative of the formula

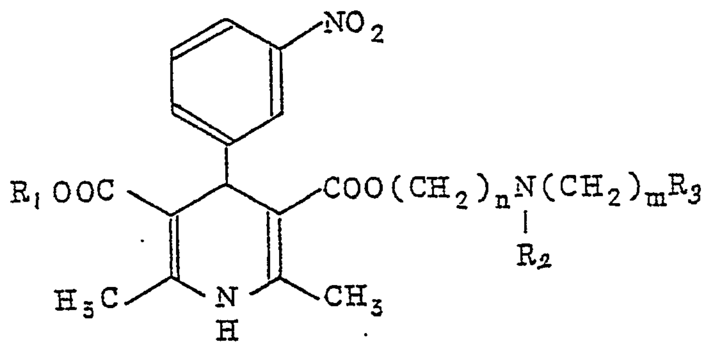

(I)

wherein $R_1$, $R_2$, $R_3$, n and m are as defined in claim 1, a salt thereof, and a pharmacologically acceptable carrier.

**Patentansprüche**

1. 1,4-Dihydropyridinderivat der Formel

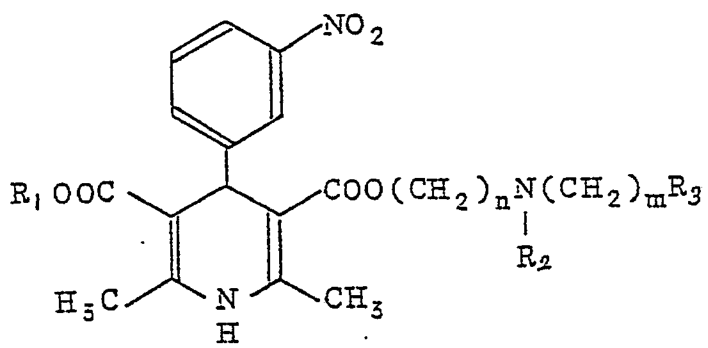

(I)

wobei $R_1$ für Cyclopropylmethyl steht, $R_2$ für Methyl steht, $R_3$ für 2,4-Dichlorphenyl steht, n für 2 steht und m für 1 steht, oder
$R_1$ für Cyclopropylmethyl steht, n für 2 steht, m für 1 steht und
$R_2$ und $R_3$ miteinander verbunden sind und mit dem benachbarten $-N(CH_2)m$-Rest für einen 5-Chlor-isoindolin-2-yl-Rest stehen, oder ein Salz desselben.

2. Verfahren zur Herstellung eines 1,4-Dihydropyridinderivats der Formel

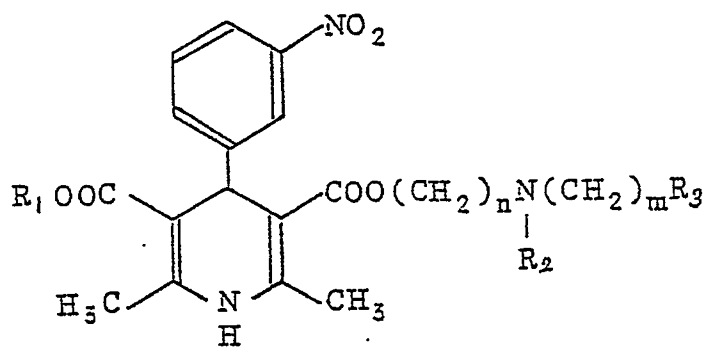

(I)

wobei $R_1$, $R_2$, $R_3$, n und m wie in Anspruch 1 definiert sind, oder eines Saltes desselben, umfassend eine Reaktionsstufe, bei der in Gegenwart von Ammoniak oder einem Salz desselben eine Verbindung der Formel

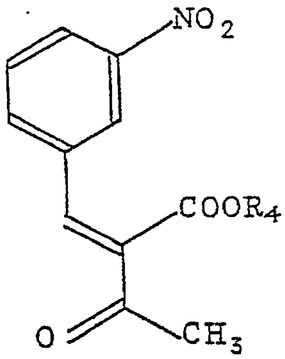

(II)

wobei $R_4$ eine Cyclopropylmethylgruppe oder einen Rest der folgenden Formel bedeutet

$$-(CH_2)_n\underset{\substack{|\\R_2}}{N}(CH_2)_mR_3$$

wobei $R_2$, $R_3$, n und m die angegebenen Bedeutungen haben, umsetzt mit einer Verbindung der Formel

$$CH_3\overset{\overset{O}{\|}}{C}CH_2COOR_5 \qquad (III)$$

wobei $R_5$ eine Cyclopropylmethylgruppe oder den Rest der Formel

$$-(CH_2)_n\underset{\substack{|\\R_2}}{N}(CH_2)_mR_3$$

bedeutet, wobei $R_2$, $R_3$, n und m die angegebenen Bedeutungen haben, mit der Maßgabe, daß $R_5$ in dieser Verbindung (III) und $R_4$ in der Verbindung (II) nicht gleichzeitig für den Rest der Formel

$$-(CH_2)_n\underset{\substack{|\\R_2}}{N}(CH_2)_mR_3$$

stehen und, falls erforderlich, die Umwandlung des resultierenden Reaktionsprodukts in das Salz.

3. Pharmazeutische Zusammensetzung zur Verbesserung des cerebralen Blutkreislaufs, umfassend eine wirksame Menge des 1,4-Dihydropyridinderivats der Formel

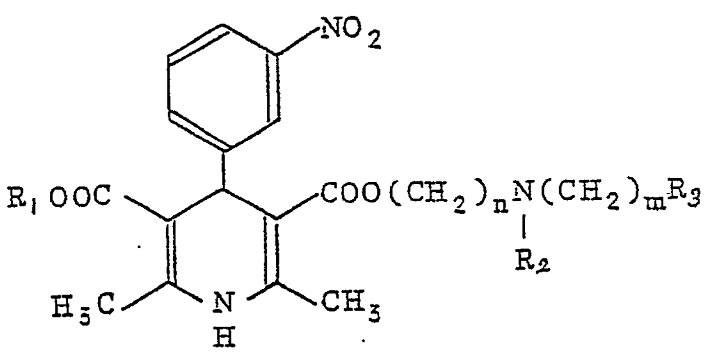

(I)

wobei $R_1$, $R_2$, $R_3$, n und m wie in Anspruch 1 definiert sind, oder eines Salzes desselben, und eine pharmazeutisch akzeptablen Träger.

**Revendications**

1. Dérivé de dihydro-1,4 pyridine de formule:

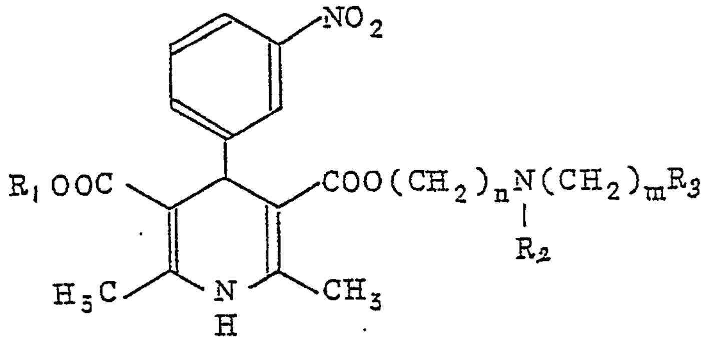

(I)

dans laquelle:

- $R_1$ représente cyclopropylméthyle;
- $R_2$ représente méthyle;
- $R_3$ représente dichloro-2,4 phényle;
- n vaut 2; et
- m vaut 1, ou
- $R_1$ représente cyclopropylméthyle;
- n vaut 2;
- m vaut 1; et
- $R_2$ et $R_3$ sont reliés pour former, conjointement avec le radical voisin $-N(CH_2)m-$, un radical chloro-5 isoindolinyle-2,

ou un sel de ce dérivé.

2. Procédé de fabrication d'un dérivé de dihydro-1,4 pyridine de formule:

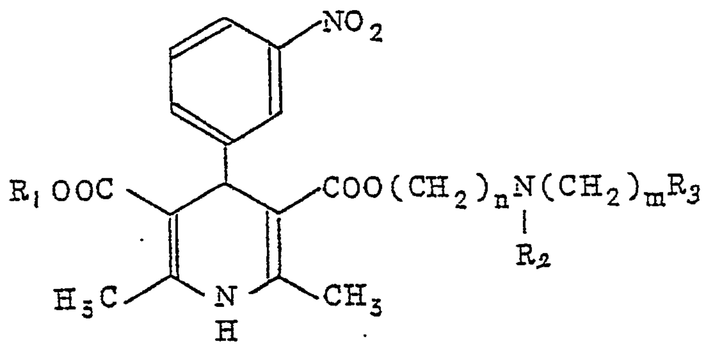

(I)

dans laquelle $R_1$, $R_3$, n et m sont tels que définis à la revendication 1,

ou d'un sel de celui-ci, qui comprend une étape de réaction, en présence d'ammoniaque ou d'un sel de cette dernière, d'un composé de formule:

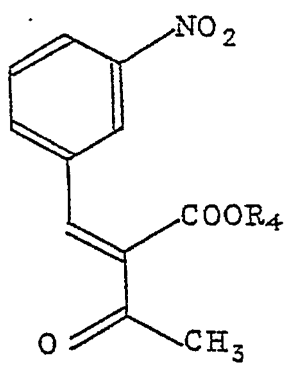

(II)

dans laquelle:

- $R_4$ représente un groupe cyclopropylméthyle ou un radical de formule

$$-(CH_2)nN(CH_2)mR_3$$
$$R_2$$

où $R_2$, $R_3$, n et m ont les significations telles que mentionnées ci-dessus, avec un composé de formule:

$$CH_3\overset{O}{\overset{\|}{C}}CH_2COOR_5 \qquad (III)$$

dans laquelle:

– $R_5$ représente un groupe cyclopropylméthyle ou le radical de formule

$$-(CH_2)nN(CH_2)mR_3$$
$$R_2$$

où $R_2$, $R_3$, n et m ont les significations telles que mentionnées ci-dessus, $R_5$ dans ce composé (III) et $R_4$ dans le composé (II) n'ayant cependant pas simultanément la même signification de radical de formule

$$-(CH_2)nN(CH_2)mR_3$$
$$R_2$$

et, si nécessaire, la conversion du produit réactionnel résultant en un sel.

3. Composition pharmaceutique pour améliorer la circulation du flux sanguin cérébral, qui comprend une quantité efficace du dérivé de dihydro-1,4 pyridine de formule:

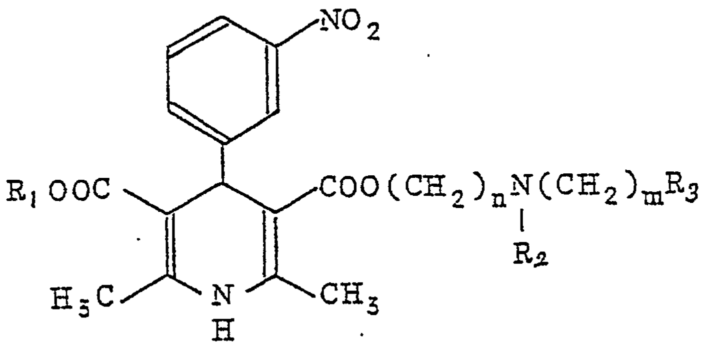

(I)

dans laquelle $R_1$, $R_2$, $R_3$, n et m sont tels que définis à la revendication 1, ou un sel de celui-ci, et un support pharmacologiquement acceptable.